# EUROPEAN PATENT SPECIFICATION

(11) **EP 4 389 124 B1**
(45) Date of publication and mention of the grant of the patent: **21.10.2026**
(21) Application number: 22858806.7
(22) Date of filing: 19.08.2022
(51) Int. Cl.: A61K 31/439, A61P 29/00, A61P 17/00, A61K 35/28, A61K 38/13, A61K 31/436

(54) **TREATMENT OF IMMUNE DISEASES BY SEQUENTIAL USE OF PIMECROLIMUS AND MESENCHYMAL STEM CELLS**
BEHANDLUNG VON IMMUNERKRANKUNGEN DURCH SEQUENZIELLE ANWENDUNG VON PIMECROLIMUS UND MESENCHYMALEN STAMMZELLEN
TRAITEMENT DE MALADIES IMMUNITAIRES PAR UTILISATION SÉQUENTIELLE DU PIMÉCROLIMUS ET DES CELLULES SOUCHES MÉSENCHYMATEUSES

(30) Priority: 19.08.2021 KR 20210109615
(43) Date of publication of application: 26.06.2024
(73) Proprietor: Kangstem Biotech Co., Ltd., Seoul 06179 (KR)
(72) Inventor: KANG, Kyung-sun, Seoul 06338 (KR); SHIN, Nari, Seoul 08832 (KR); JUNG, Namhee, Seoul 07269 (KR); LEE, Seunghee, Seoul 08797 (KR); PARK, Hwanhee, Seoul 07516 (KR); PARK, Soyoung, Seoul 08325 (KR)
(74) Representative: Zwicker, Jörk
(86) International application number: PCT/KR2022/012435
(87) International publication number: WO 2023/022569

(56) References cited:
- WO-A1-2017/204639
- WO-A1-2020/247385
- WO-A1-2021/022377
- WO-A2-2019/012334
- KR-A- 20130 096 661
- US-A1- 2012 164 114
- US-A1- 2018 214 489
- ANONYMOUS OMAR A ET AL: "Calcineurin Inhibitors - StatPearls - NCBI Bookshelf", 12 November 2023 (2023-11-12), XP093352274, Retrieved from the Internet <URL:https://www.ncbi.nlm.nih.gov/books/NBK558995/>
- HAJKOVA MICHAELA, JABUREK FILIP, PORUBSKA BIANKA, BOHACOVA PAVLA, HOLAN VLADIMIR, KRULOVA MAGDALENA: "Cyclosporine A promotes the therapeutic effect of mesenchymal stem cells on transplantation reaction", CLINICAL SCIENCE, vol. 133, no. 21, 15 November 2019 (2019-11-15), GB , pages 2143 - 2157, XP093036254, ISSN: 0143-5221, DOI: 10.1042/CS20190294
- CHEN YINGYU, YAN GUOLIANG, MA YUNHAN, ZHONG MENGYA, YANG YAN, GUO JUNJUN, WANG CHENXI, HAN WEIMIN, ZHANG LIYI, XU SHUANGYUE, HUANG: "Combination of mesenchymal stem cells and FK506 prolongs heart allograft survival by inhibiting TBK1/IRF3-regulated-IFN-γ production", IMMUNOLOGY LETTERS, vol. 238, 1 October 2021 (2021-10-01), NL , pages 21 - 28, XP093036256, ISSN: 0165-2478, DOI: 10.1016/j.imlet.2021.06.007
- PARK HWAN HEE, LEE SEUNGHEE, YU YEONSIL, YOO SAE MI, BAEK SONG YI, JUNG NAMHEE, SEO KWANG-WON, KANG KYUNG-SUN: "TGF-β secreted by human umbilical cord blood-derived mesenchymal stem cells ameliorates atopic dermatitis by inhibiting secretion of TNF-α and IgE", STEM CELLS, vol. 38, no. 7, 1 July 2020 (2020-07-01), pages 904 - 916, XP093036258, ISSN: 1066-5099, DOI: 10.1002/stem.3183
- SHIN NARI, JUNG NAMHEE, LEE SEUNG-EUN, KONG DASOM, KIM NAM GYO, KOOK MYUNG GEUN, PARK HWANHEE, CHOI SOON WON, LEE SEUNGHEE, KANG K: "Pimecrolimus interferes the therapeutic efficacy of human mesenchymal stem cells in atopic dermatitis by regulating NFAT-COX2 signaling", STEM CELL RESEARCH & THERAPY, vol. 12, no. 1, 1 December 2021 (2021-12-01), London, UK , XP093036261, ISSN: 1757-6512, DOI: 10.1186/s13287-021-02547-8

## Description

### [Technical Field]

The present invention relates to the prevention or treatment of an immune disease caused by excessive immunity by means of pimecrolimus and mesenchymal stem cells.

The invention is defined in the claims.

### [Background Art]

Immune disease is a disease caused by damage or abnormal function of the body's immune system due to various causes, such as exogenous factors, genetic factors, endogenous factors, *etc.,* and is characterized by a significant decrease in immunity or a rapid increase in immunity. In particular, unlike the cases where the immunity is reduced, in which pathological symptoms can be relatively predicted, the cases of a rapid increase in immunity are accompanied by various unpredictable pathological symptoms, and thus, research related to the increased immunity is actively underway.

Atopic dermatitis (AD), which is one of the immune diseases caused by excessive immunity in which the immunity is rapidly increased, is a chronic and relapsing skin disorder characterized by eczematous skin lesions with severe pruritus and epidermal barrier dysfunction. The pathogenesis of AD is characterized by excessive type 2 helper T (Th2) cell-mediated inflammation, resulting in increased serum levels of total IgE and skin barrier dysfunction. The basic management of AD consists of epidermal barrier repair with emollients and anti-inflammatory drugs such as corticosteroids or calcineurin inhibitors. Calcineurin inhibitors are one of the most common medications prescribed for patients with AD. These drugs bind to FK506-binding protein-12 (FKBP-12), inhibit the serine/threonine phosphatase calcineurin, and subsequently prevent nuclear translocation of nuclear factor of activated T cells (NFAT). Consequently, NFAT-mediated transcription of proinflammatory cytokines in T cells is suppressed, thereby improving AD.

Currently, mesenchymal stem cell (MSC)-based therapy has appeared to be as a promising approach to alleviate AD. MSCs are capable of self-renewing and able to differentiate into multiple lineages for damaged tissue regeneration. Additionally, MSCs have been considered as an immunomodulator through the regulation of the proliferation, recruitment and function of innate and adaptive immune cells. Various methods of isolating MSCs, stem cell products, or stromal cells with anti-inflammatory potential have been described and it has been suggested that combination with other anti-inflammatory drugs such as corticosteroids or calcineurin inhibitors might enhance their effect in treating diseases caused by excessive immunity(US2012/164114 A1; US 2018/214489 A1; WO 2019/012334 A2; WO 2020/247385 A1; WO 2021022377). Further, MSCs are expected to have a therapeutic effect on AD by inhibiting mast cell degranulation through prostaglandin E₂ (PGE₂) and TGF-beta (Korean Laid-Open Publication Nos. 10-2009-0111269, 10-2010-0104385, Korean Patent No. 10-1070730, *etc*.); however, their specific therapeutic effect on AD has not been proven in practical clinical trials.

### [Disclosure]

### [Technical Problem]

The present inventors have made extensive efforts to develop a method for effectively treating atopic dermatitis using stem cells. As a result, it was confirmed that when mesenchymal stem cells and pimecrolimus used sequentially, the immunosuppressive activity was increased, compared to when the mesenchymal stem cells and pimecrolimus were used independently, thereby completing the present invention.

### [Technical Solution]

The object of the present invention is to provide a pharmaceutical combination for use preventing or treating an immune disease, including: a first composition containing pimecrolimus or a pharmaceutically acceptable salt thereof; and a second composition containing mesenchymal stem cells, wherein the first composition and the second composition contained in the pharmaceutical combination are separately, and sequentially administered to an individual having an immune disease caused by excessive immunity.

### [Advantageous Effects]

When the pharmaceutical combination including pimecrolimus and mesenchymal stem cells provided in the present invention is used, a significantly higher level of immunosuppressive activity than that obtained using conventional calcineurin inhibitors or mesenchymal stem cells can be obtained. Therefore, it can be used to more effectively prevent or treat immune diseases caused by excessive immunity.

### [Brief Description of Drawings]

FIG. 1 is a graph showing the results of comparing the severity of skin lesions in atopic dermatitis animal model treated with pimecrolimus or mesenchymal stem cells, where MSC represents the stem cell-only administration group, Pime represents the pimecrolimus-only administration group, and MSC+Pime represents the combined administration group of stem cells and pimecrolimus.
FIG. 2a is a graph comparing the change in the cell viability of stem cells according to the treatment concentrations of pimecrolimus.
FIG. 2b is a graph comparing the change in the proliferative capacity of stem cells according to the treatment concentrations of pimecrolimus.
FIG. 2c is a graph comparing the change in the cell cycle profile of stem cells according to the treatment concentrations of pimecrolimus.
FIG. 2d is a graph comparing the change in the cellular morphology of stem cells according to the treatment concentrations of pimecrolimus.
FIG. 2e is a graph comparing the change in the expression levels of surface markers (CD45, CD34, CD36, CD105, CD29, CD73) of stem cells according to pimecrolimus treatment.
FIG. 3 is a graph showing the results of analyzing the effect of pimecrolimus on the immunosuppressive activity of stem cells on PBMC cells treated with ConA.
FIG. 4a is a graph showing the results of comparing the level of TGF-β expressed in stem cells according to the treatment concentrations of pimecrolimus.
FIG. 4b is a graph showing the results of comparing the level of PGE₂ expressed in stem cells according to the treatment concentrations of pimecrolimus.
FIG. 4c is an image of Western blot showing the results of comparing the level of COX2 expressed from stem cells according to the treatment concentrations of pimecrolimus and a graph analyzing its density.
FIG. 5a is a graph comparing the immunosuppressive activity obtained by treating stem cells and pimecrolimus under the same conditions.
FIG. 5b is a graph comparing the immunosuppressive activity obtained by treating pimecrolimus first, followed by stem cells.
FIG. 5c is a graph comparing the immunosuppressive activity obtained by treating stem cells first, followed by pimecrolimus.
FIG. 5d is a graph showing the results of analyzing the change in immunosuppressive activity according to the treatment order when stem cells and pimecrolimus were treated in combination.

### [Detailed Description of Preferred Embodiments]

In order to achieve the above-described objects, the present invention provides a pharmaceutical combination for use preventing or treating an immune disease caused by excessive immunity, including separately:
(a) a first composition containing pimecrolimus or a pharmaceutically acceptable salt thereof; and
(b) a second composition containing mesenchymal stem cells;
wherein the first and second composition are administered sequentially.

As used herein, the term "calcineurin inhibitor" generally refers to an immunomodulatory substance produced during the fermentation process of microorganisms, and is known to have an effect of mainly suppressing the response of T cells. Functionally, the calcineurin inhibitor is known to inhibit the expression of intracellular cytokine genes by inhibiting the reaction of calcineurin, a type of phosphorylation enzyme, and consequently reduces the secretion of various inflammatory cytokines.

The calcineurin inhibitor provided in the present invention is pimecrolimus.

As used herein, the term "pharmaceutically acceptable salt" refers to a formulation which does not damage biological activities and properties of the calcineurin inhibitor. The pharmaceutically acceptable salt may include acid addition salts with acids capable of forming a non-toxic acid addition salt containing pharmaceutically acceptable anions, *e.g.,* an inorganic acid such as hydrochloric acid, sulfuric acid, nitric acid, phosphoric acid, hydrobromic acid, hydroiodic acid, *etc.;* an organic carboxylic acid such as tartaric acid, formic acid, citric acid, acetic acid, trichloroacetic acid, trifluoroacetic acid, gluconic acid, benzoic acid, lactic acid, fumaric acid, maleic acid, salicylic acid, *etc.;* or a sulfonic acid such as methanesulfonic acid, ethanesulfonic acid, benzenesulfonic acid, *p*-toluenesulfonic acid, *etc.* For example, the pharmaceutically acceptable carboxylic acid salts include metal salts or alkaline earth metal salts formed by lithium, sodium, potassium, calcium, magnesium, *etc.;* amino acid salts such as lysine, arginine, guanidine, *etc.;* organic salts such as dicyclohexylamine, *N*-methyl-D-glucamine, tris(hydroxymethyl)methylamine, diethanolamine, choline, triethylamine, *etc.* In the case of amlodipine, a commercially available besylic acid salt is preferable.

As used herein, the term "stem cell" refers to a cell having excellent proliferation ability and is capable of being differentiated into various body tissues.

The stem cells provided in the present invention are mesenchymal stem cells, such as adipose-derived mesenchymal stem cells, umbilical cord blood-derived mesenchymal stem cells, umbilical cord-derived mesenchymal stem cells, cardiac muscle-derived mesenchymal stem cells, placenta-derived mesenchymal stem cells, cartilage-derived mesenchymal stem cells, and bone marrow-derived mesenchyme, *etc.* In still another example, the various mesenchymal stem cells may be used alone or in various combinations.

As used herein, the term "immune disease" refers to a disease caused by damage or abnormal function of the body's immune system due to various causes such as exogenous factors, genetic factors, endogenous factors, *etc.*

In the present invention, the immune disease is an immune disease caused by excessive immunity such as an allergic immune disease or an autoimmune disease caused by excessive immunity. In another example, it may be an autoimmune disease, such as Graves' disease, rheumatoid arthritis, Hashimoto's thyroiditis, systemic lupus erythematosus (lupus), vasculitis, Addison's disease, polymyositis, Sjögren's syndrome, progressive systemic sclerosis, *etc.*

As used herein, the term "prevention" refers to all actions that suppress or delay the occurrence of an immune disease by administration of the pharmaceutical composition provided in the present invention.

As used herein, the term "treatment" refers to all actions that alleviate or beneficially change the symptoms of an individual that is suspicious for the occurrence of an immune disease or the occurrence of an immune disease is confirmed by administration of the pharmaceutical composition.

The pharmaceutical combination provided for use in the present invention separately includes a first composition containing pimecrolimus or a pharmaceutically acceptable salt thereof and a second composition containing mesenchymal stem cells thereof, and the first and second composition are administered sequentially. When the pimecrolimus and stem cells are used simultaneously, the immunomodulatory activity exhibited by these active ingredients is reduced due to mutual interference. Therefore, the pharmaceutical combination provided in the present invention is designed to contain these active ingredients in an isolated form as two different compositions, separately containing each ingredient. Additionally, the terms "first composition" and "second composition" do not imply an order for the preparation or administration.

The calcineurin inhibitor or a pharmaceutically acceptable salt thereof contained in the first composition may be included in an amount of 0.0001 to 50% by weight, specifically 0.01 to 10% by weight, based on the weight of the total composition, but is not limited thereto.

The stem cells contained in the second composition may be included in an amount of 1x10⁵ to 9x10⁹ cells/ml, specifically, 5x10⁵ to 5x10⁹ cells/ml, and more specifically 1x10⁶ to 1x10⁹ cells/ml, but is not limited thereto.

Additionally, the first composition and the second composition may further include a pharmaceutically acceptable carrier, excipient or diluent commonly used in the preparation of the pharmaceutical compositions independently, and the carrier may include a non-naturally occurring carrier. The carriers, excipients, and diluents may include cryoprotectants, lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and minerals.

Specifically, the pharmaceutical combination may be formulated with commonly used diluents or excipients, such as fillers, extenders, binders, wetting agents, disintegrants, surfactants, *etc.* Solid formulations for oral administration may include tablets, pills, powders, granules, capsules, *etc.,* but are not limited thereto. Such solid formulations may be prepared by mixing with at least one excipient, for example, starch, calcium carbonate, sucrose, lactose, gelatin, *etc.* In addition to simple excipients, lubricants such as magnesium stearate or talc may also be used. Liquid formulations for oral administration may be prepared by adding various excipients, for example, wetting agents, flavoring agents, aromatics, preservatives, *etc.,* in addition to liquid paraffin. Formulations for parenteral administration may include sterile aqueous solutions, nonaqueous solvents, suspensions, emulsions, lyophilized preparations, and suppositories. The non-aqueous solutions and the suspensions may include propylene glycol, polyethylene glycol, vegetable oil such as olive oil, injectable ester such as ethyloleate, *etc.* The base for suppositories may include witepsol, macrogol, tween 61, cacao butter, laurin butter, glycerogelatin, *etc.*

The administration of mesenchymal stem cells to an atopic dermatitis animal model has the effect of alleviating atopic skin lesions, but when the mesenchymal stem cells were administered in combination with pimecrolimus, a type of calcineurin inhibitor, the therapeutic efficacy was reduced compared to when treated with stem cells alone, and the reasons behind the result were investigated.

To this end, the present inventors studied the effects of pimecrolimus on stem cells, and as a result, pimecrolimus did not have any effect on various factors, such as cell viability, proliferative capacity, cell cycle profile, or expression levels of surface markers, *etc.,* but inhibited the expression of prostaglandin E₂ (PGE₂) by inhibiting the expression of COX2 expressed in stem cells, thereby reducing the immunosuppressive activity of stem cells.

Accordingly, various studies have been conducted to develop a method to increase immunosuppressive activity by treating pimecrolimus and mesenchymal stem cells in combination. As a result, it was confirmed that pimecrolimus and stem cells may result in large differences in immunosuppressive activity depending on the combination treatment method. That is, it was confirmed that pimecrolimus may interfere with the immunosuppressive activity of stem cells, and that stem cells may also affect the immunosuppressive activity of pimecrolimus. Therefore, it was confirmed that when stem cells and pimecrolimus were treated sequentially, the immunosuppressive activity showed a synergistic effect.

Specifically, it was confirmed that when pimecrolimus is first treated and stem cells are additionally treated after a certain time has elapsed, or stem cells are first treated and pimecrolimus is additionally treated after a certain time has elapsed, the immunosuppressive activity can be significantly increased, and the most efficient immunosuppressive activity can be obtained by first treating pimecrolimus and then further treating stem cells after a certain period of time.

In particular, the definitions of calcineurin inhibitor, stem cells, immune diseases, prevention and treatment are as described above.

As used herein, the term "administration" to any action of introducing a predetermined substance into an individual by an appropriate method.

As used herein, the term "individual" refers to any animal, such as rats, mice, and livestock, including humans that may or is likely to have the immune disease. In a specific example, it may be mammals, including humans.

In one example, the first composition may be first administered once or multiple times, and the second composition may be additionally administered once or multiple times. Additionally, in another example, the first composition or the second composition may be additionally administered individually once or multiple times, after administering the second composition.

In addition, in still another example, the second composition may be first administered once or multiple times, and the first composition may be additionally administered once or multiple times. Further, in yet another example, the first composition or the second composition may be additionally administered individually once or multiple times, after administering the first composition.

Additionally, the method of administering the first composition and the second composition may be performed once or repeatedly. When performed repeatedly, the frequency of administration of the first composition and the frequency of administration of the second composition may be the same or different.

In one example, the administration may be realized in the order of the first composition, the second composition, and the first composition, or the second composition, the first composition, and the second composition. In another example, the administration may be realized in the order of the first composition, the second composition, the first composition, and the second composition, or the second composition, the first composition, the second composition and the first composition. In still another example, the administration may be realized in the order of the first composition, the second composition, the first composition, the second composition, and the first composition, or the second composition, the first composition, the second composition, the first composition, and the second composition. In yet another example, the administration may be realized in the order of the first composition, the second composition, the first composition, the second composition, the first composition, and the second composition, or the second composition, the first composition, the second composition, the first composition, the second composition, and the first composition. In even another example, the first composition may be administered once or multiple times, subsequently, the second composition may be administered once or multiple times, and then the first composition may be administered once or multiple times, and the reverse order may also be possible.

As used herein, the term "pharmaceutically effective amount" means an amount which is sufficient to treat diseases at a reasonable benefit/risk ratio applicable to any medical treatment without causing any adverse effects, and the level of the effective dose may be easily determined by those of ordinary skill in the art based on the factors including a patient's gender, age, body weight and health condition, type of disease(s), severity of illness, drug activity, drug sensitivity, administration method, administration time, administration route and excretion rate, length of treatment, factors including drug(s) to be mixed or concurrently used in combination, and other factors well known in the medical field.

Specifically, the pharmaceutical composition of the present invention may be administered in a daily dosage of 0.0001 to 1000 mg/kg, and more specifically 0.001 to 100 mg/kg, based on the solid content, and the dose may be administered once per day or in several divided doses per day.

The routes and methods for administration of the composition are not particularly limited, and the administration may be realized by any routes and methods so long as the composition can reach desired areas. Specifically, the composition may be administered by various oral or parenteral routes, and non-limiting examples of the route of administration may include subcutaneous, oral, rectal, topical, intravenous, intraperitoneal, intramuscular, intra-arterial, transdermal, intranasal, or inhalation, *etc.*

### [Mode for Carrying Out the Invention]

Hereinafter, the present invention will be described in detail by way of Examples. However, these Examples are merely preferred Examples given for illustrative purposes, and thus, the scope of the invention is not intended to be limited to or by these Examples.

### Example 1: Effect of Pimecrolimus and Mesenchymal Stem Cells on Atopic Dermatitis Animal Model

First, an atopic dermatitis animal model was constructed.

Specifically, the hair of NC/Nga mice (male, seven-week-old) was removed from the lower part of the ear to the upper part of the tail and, 100 mg/head of Df *(Dermatophagoides farinae;* Biostir-AD, Biostir, Kobe, Japan) was applied evenly with a cotton swab to the depilated back and the back of the ear twice a week, for three weeks (total of six times), thereby constructing the atopic dermatitis animal model.

Next, pimecrolimus, known as a calcineurin inhibitor, and mesenchymal stem cells (hUCB-MSC) were administered individually or in combination to the animal model, and the changes in skin lesions were analyzed for 7 days.

Specifically, Df was finally induced, and the pimecrolimus or hUCB-MSC were administered on the 3rd day after the induction to the skin lesion areas treated with Df. Pimecrolimus was administered once a day for 7 days at a dose of 0.2g/head, and 2 × 10⁶ hUCB-MSCs were subcutaneously injected. In particular, mice, in which atopic dermatitis was not induced, were used as the negative control group (NC), and mice that were not treated with pimecrolimus or hUCB-MSC after induction of atopic dermatitis were used as the positive control group (PC).

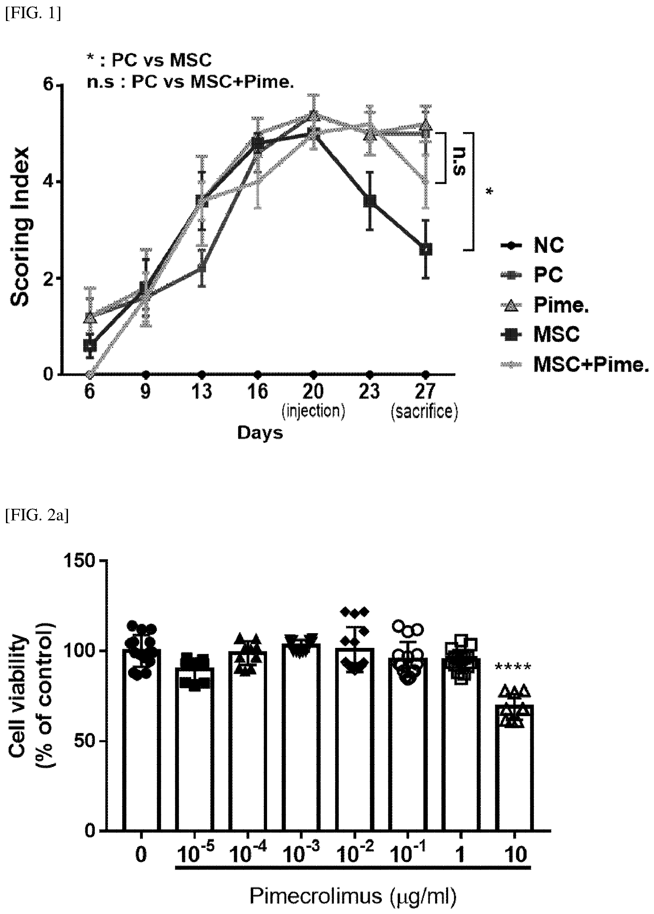

The change in the skin lesions were analyzed by measuring the severity of the skin lesions and comparing them, which was reported for four symptoms: erythema, scarring/dryness, edema, and erosion and scored from 0 to 3 points (0: none, 1: mild, 2: moderate and 3: severe) (FIG. 1)

FIG. 1 is a graph showing the results of comparing the severity of skin lesions in atopic dermatitis animal model treated with pimecrolimus or hUCB-MSC, where MSC represents the hUCB-MSC-only administration group, Pime represents the pimecrolimus-only administration group, and MSC+Pime represents the combined administration group of hUCB-MSC and pimecrolimus.

As shown in FIG. 1, it was confirmed that when hUCB-MSC were administered alone, the lesions of atopic dermatitis were alleviated, but when hUCB-MSC and pimecrolimus were administered in combination, the effect of alleviating the lesions of atopic dermatitis was reduced.

From the above results, it was confirmed that pimecrolimus inhibited the AD therapeutic effect of hUCB-MSC, which was contrary to the expected result of a synergistic effect between hUCB-MSC and calcineurin inhibitor, known as a therapeutic agent for atopic dermatitis.

### Example 2: Analysis of Effect of Pimecrolimus on Stem Cells

Assuming that the results obtained in Example 1 were the result of pimecrolimus affecting stem cells, it was analyzed what effect pimecrolimus has on hUCB-MSC themselves.

First, the stem cells (hUCB-MSC) were cultured and treated with various concentrations of pimecrolimus for 3 days, and then the change in cell viability of hUCB-MSC were compared using the MTT assay (FIG. 2a).

FIG. 2a is a graph comparing the change in cell viability of hUCB-MSC according to the treatment concentrations of pimecrolimus.

As shown in FIG. 2a, it was confirmed that pimecrolimus in the range of 0.1 to 1 Fg/ml did not change the cell viability of hUCB-MSC.

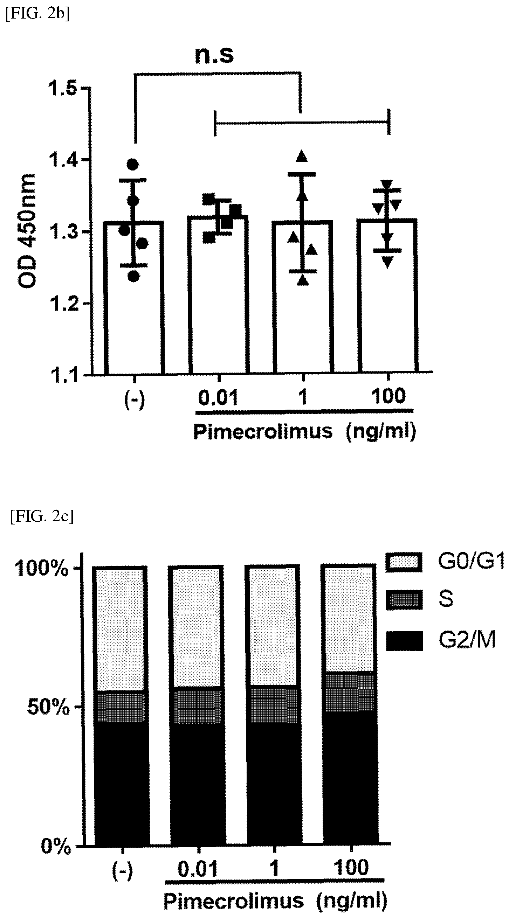

Next, the stem cells (hUCB-MSC) were cultured and treated with various concentrations of pimecrolimus for 3 days, and then the change in proliferative capacity of hUCB-MSC were compared (FIG. 2b).

FIG. 2b is a graph comparing the change in proliferative capacity of hUCB-MSC according to the treatment concentrations of pimecrolimus.

As shown in FIG. 2b, it was confirmed that pimecrolimus in the range of 0.01 to 100 ng/ml did not change the proliferative capacity of hUCB-MSC.

Next, the stem cells (hUCB-MSC) were cultured and treated with various concentrations of pimecrolimus for 3 days, and then the change in cell cycle profile of hUCB-MSC were compared using cell cycle analysis (FIG. 2c).

FIG. 2c is a graph comparing the change in cell cycle profile of hUCB-MSC according to the treatment concentrations of pimecrolimus.

As shown in FIG. 2c, it was confirmed that pimecrolimus in the range of 0.01 to 100 ng/ml did not change the cell cycle profile of hUCB-MSC.

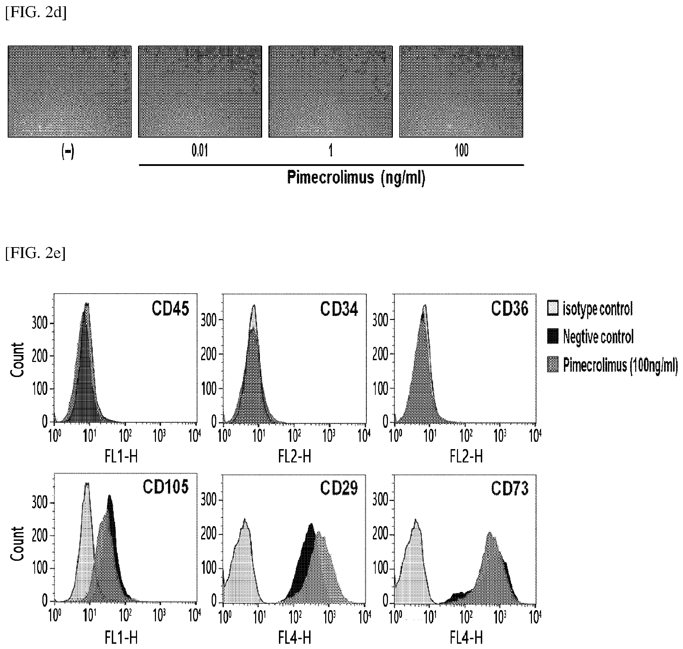

Next, the stem cells (hUCB-MSC) were cultured and treated with various concentrations of pimecrolimus for 3 days, and then the change in cellular morphology of hUCB-MSC were compared (FIG. 2d).

FIG. 2d is a graph comparing the change in cellular morphology of hUCB-MSC according to the treatment concentrations of pimecrolimus.

As shown in FIG. 2d, it was confirmed that pimecrolimus in the range of 0.01 to 100 ng/ml did not change the cellular morphology of hUCB-MSC.

Lastly, the stem cells (hUCB-MSC) were cultured and treated with 100 ng/ml of pimecrolimus for 3 days, and then the expression levels of surface markers (CD45, CD34, CD36, CD105, CD29, CD73) of hUCB-MSC were compared using flow cytometry. (FIG. 2e).

FIG. 2e is a graph comparing the change in the expression levels of surface markers (CD45, CD34, CD36, CD105, CD29, CD73) of hUCB-MSC according to pimecrolimus treatment.

As shown in FIG. 2e, it was confirmed that pimecrolimus at 100 ng/ml did not change the expression levels of surface markers of hUCB-MSC.

In summary of the above results, it was found that pimecrolimus had no effect on hUCB-MSC themselves.

### Example 3: Analysis of Effect of Pimecrolimus on Immunomodulatory Ability of hUCB-MSC

From the results of Example 2, it was confirmed that pimecrolimus had no effect on hUCB-MSC themselves. Therefore, it was analyzed whether pimecrolimus interfered with the immunomodulatory ability of hUCB-MSC.

### Example 3-1: MLR Analysis

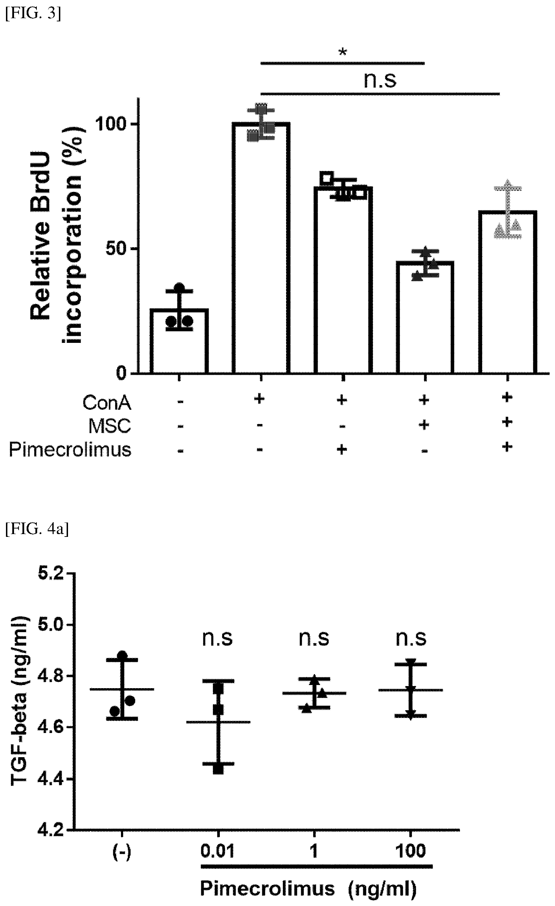

PBMC cells treated with Concanavalin A (ConA) were cultured and treated with pimecrolimus and mesenchymal stem cells (hUCB-MSC) individually or in combination, and then the immunosuppressive activity measured in each experimental group was compared (FIG. 3). In particular, PBMC cells that were not treated with ConA, not co-cultured with hUCB-MSC, and not treated with pimecrolimus were used as the negative control (NC), and as the positive control (PC) treated with ConA, PBMC cells that were not co-cultured with hUCB-MSC and not treated with pimecrolimus were used as the positive control (PC).

FIG. 3 is a graph showing the results of analyzing the effect of pimecrolimus on the immunosuppressive activity of hUCB-MSC on PBMC cells treated with ConA.

As shown in FIG. 3, it was confirmed that the immune activity was increased in the positive control group treated with ConA, but the immune activity was suppressed by treatment with hUCB-MSC or pimecrolimus. However, it was confirmed that the immunosuppressive activity observed when hUCB-MSC were treated alone was reduced by treatment with pimecrolimus.

### Example 3-2: Analysis of Expression Levels of Immune Activity Regulators

Since it was confirmed from the results of Example 3-1 that the immunosuppressive activity of hUCB-MSC was suppressed by pimecrolimus, it was confirmed whether the expression level of TGF-β or prostaglandin E₂ (PGE₂), which is an immune activity regulator expressed in stem cells, was affected by pimecrolimus.

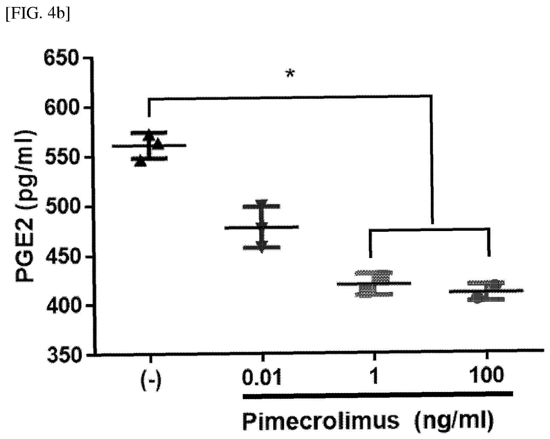

Specifically, hUCB-MSCs were seeded in a 6-well cell culture plate at a density of 1.5 × 10⁵ cells per well. After 24 hours of incubation, hUCB-MSCs were treated with pimecrolimus at various concentrations and further incubated for 72 hours. After the incubation was completed, the supernatant was collected by centrifugation and the thus-obtained supernatant was applied to Human TGF-β1 Quantikine ELISA (R&D Systems, Minneapolis, MN, USA) and Human PGE₂ Quantikine ELISA (R&D Systems) to analyze the expression level of TGF-β or prostaglandin E₂ (PGE₂) (FIGS. 4a and 4b).

FIG. 4a is a graph showing the results of comparing the level of TGF-β expressed in hUCB-MSCs according to the treatment concentrations of pimecrolimus.

As shown in FIG. 4a, it was confirmed that the level of TGF-β expressed in hUCB-MSCs treated with pimecrolimus in the range of 0.01 to 100 ng/ml did not show a significant change.

FIG. 4b is a graph showing the results of comparing the level of PGE₂ expressed in hUCB-MSCs according to the treatment concentrations of pimecrolimus.

As shown in FIG. 4b, it was confirmed that the level of PGE₂ expressed in hUCB-MSCs treated with pimecrolimus in the range of 0.01 to 100 ng/ml was rapidly reduced in proportion to the treatment concentrations of pimecrolimus.

### Example 3-3: Analysis of COX2 Expression Level

From the results of Example 3-2, it was confirmed that treatment of stem cells with pimecrolimus had the effect of suppressing the expression of prostaglandin E₂ (PGE₂), an immune activity regulator. To verify this, the expression level of COX2 was analyzed. That is, since it is known that PGE₂ is synthesized from arachidonic acid released from membrane phospholipids by COX (cyclooxygenase), it was confirmed whether the inhibition of PGE₂ expression was caused by the inhibition of COX expression, which was previously confirmed.

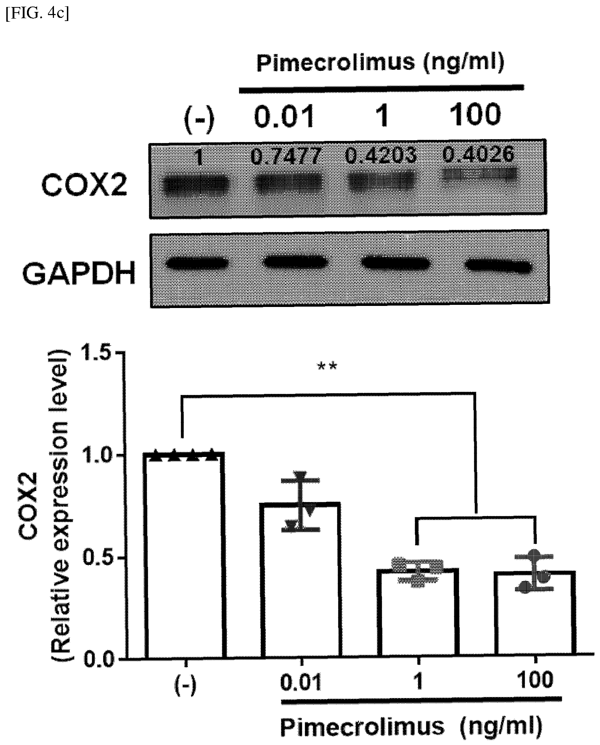

hUCB-MSCs treated with pimecrolimus were obtained according to the method of Example 3-2, centrifuged to collect the precipitated cells, and then disrupted to obtain cell lysate. Subsequently, Western blot analysis was performed using the thus-obtained cell lysate and COX2 antibodies (FIG. 4c). In particular, GAPDH was used as an internal control.

FIG. 4c is an image of Western blot showing the results of comparing the level of COX2 expressed in hUCB-MSCs according to the treatment concentrations of pimecrolimus and a graph analyzing its density.

As shown in FIG. 4c, it was confirmed that the level of PGE₂ expressed in hUCB-MSCs treated with pimecrolimus in the range of 0.01 to 100 ng/ml was rapidly reduced in proportion to the treatment concentrations of pimecrolimus.

In summary of the results of Examples 3-1 to 3-3, it was found that pimecrolimus had an effect on hUCB-MSCs to inhibit the expression of COX2, thereby suppressing the expression level of PGE₂ in hUCB-MSCs, and the immunosuppressive activity of hUCB-MSCs was reduced by the inhibition of the expression level of PGE₂.

### Example 4: Correlation Between hUCB-MSCs and Pimecrolimus on Immunosuppressive Activity

Since it was confirmed through Example 3 that pimecrolimus had an effect of reducing the immunosuppressive activity of hUCB-MSCs, various conditions were set to analyze such effect in more depth, and the results were analyzed using the MLR assay.

Specifically, PBMC cells treated with Concanavalin A (ConA) were cultured and treated with pimecrolimus and hUCB-MSC individually or in combination, and then the immunosuppressive activity measured in each experimental group was compared.

In particular, 10 experimental groups were set according to the combination pattern of pimecrolimus and hUCB-MSC (Table 1)

**[Table 1]**

| Setting of 10 Experimental Groups | |
|---|---|
| Experimental Group | Setting of Experimental Groups |
| Experimental Group 1 | Pimecrolimus and hUCB-MSCs were treated in combination for 3 to 7 days. |
| Experimental Group 2 | Pimecrolimus was treated alone for 3 to 7 days. |
| Experimental Group 3 | hUCB-MSCs were treated alone for 3 to 7 days. |
| Experimental Group 4 | Pimecrolimus was treated for 3 to 7 days, while hUCB-MSCs were treated for 5 to 7 days in combination. |
| Experimental Group 5 | hUCB-MSCs were treated alone for 5 to 7 days. |
| Experimental Group 6 | Pimecrolimus and hUCB-MSCs were treated in combination for 5 to 7 days. |
| Experimental Group 7 | hUCB-MSCs were treated for 3 to 7 days, while pimecrolimus was treated for 5 to 7 days in combination. |
| Experimental Group 8 | hUCB-MSCs were treated alone for 3 to 5 days, and then, pimecrolimus was treated alone for 5 to 7 days. |
| Experimental Group 9 | Pimecrolimus was treated alone for 5 to 7 days. |
| Experimental Group 10 | hUCB-MSCs were treated alone for 3 to 5 days. |

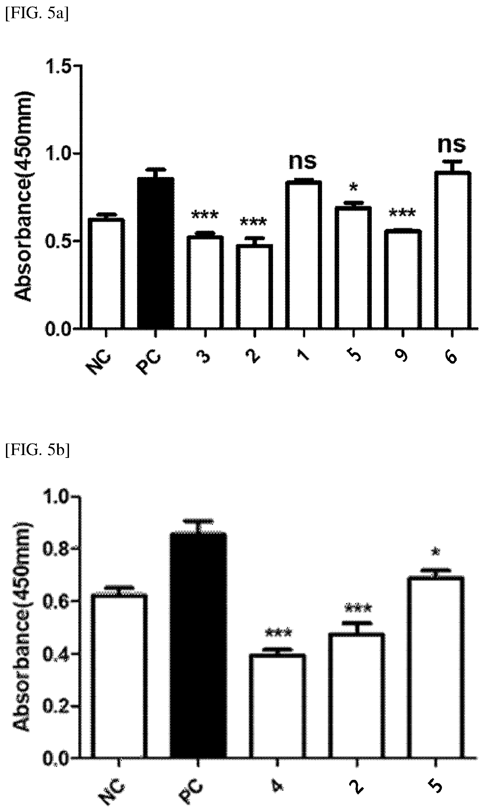

The experimental results of each experimental group were classified and analyzed according to the purpose of analysis (FIGS. 5a to 5b).

First, the results of comparing hUCB-MSCs and pimecrolimus under the same treatment conditions were analyzed (FIG. 5a).

FIG. 5a is a graph comparing the immunosuppressive activity obtained by treating hUCB-MSCs and pimecrolimus under the same conditions.

As shown in FIG. 5a, when hUCB-MSCs and pimecrolimus were treated simultaneously, no immunosuppressive activity was observed regardless of the treatment time (Experimental Groups 1 and 6).

In addition, when hUCB-MSCs and pimecrolimus were treated separately, there was some difference depending on the treatment time, but overall, the immunosuppressive activity of pimecrolimus was comparatively superior over the immunosuppressive activity of hUCB-MSCs (Experimental Group 2 and 3; Experimental Groups 5 and 9)

Next, the results of treating pimecrolimus first, followed by hUCB-MSCs were analyzed (FIG. 5b).

FIG. 5b is a graph comparing the immunosuppressive activity obtained by treating pimecrolimus first, followed by hUCB-MSCs.

As shown in FIG. 5b, when hUCB-MSCs were treated sequentially after treating pimecrolimus, the highest immunosuppressive activity was observed among the 10 experimental groups (Experimental Group 4).

These results were comparatively superior compared with the results of treating pimecrolimus and hUCB-MSCs individually under the same conditions (Experimental Groups 2 and 5), thus, it was analyzed that pimecrolimus and hUCB-MSCs show a synergistic effect.

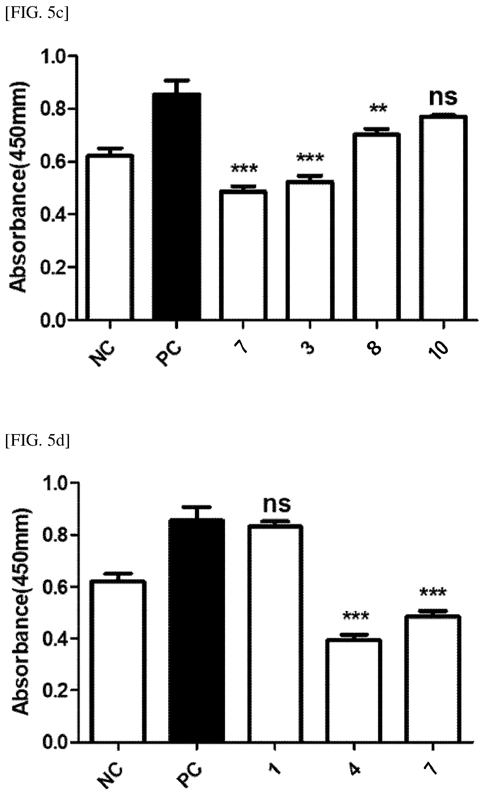

Further, the results of treating hUCB-MSCs first, followed by pimecrolimus were analyzed (FIG. 5c).

FIG. 5c is a graph comparing the immunosuppressive activity obtained by treating hUCB-MSCs first, followed by pimecrolimus.

As shown in FIG. 5c, a comparatively superior immunosuppressive activity was observed when pimecrolimus was treated sequentially after treating hUCB-MSCs, compared to when hUCB-MSCs were treated alone (Experimental Groups 3 and 7).

Moreover, a comparatively superior immunosuppressive activity was observed when hUCB-MSCs and pimecrolimus were treated sequentially, but not simultaneously, compared to when hUCB-MSCs were treated alone (Experimental Groups 8 and 10).

Lastly, the change according to the treatment order was analyzed when hUCB-MSCs and pimecrolimus were treated in combination (FIG. 5d).

FIG. 5d is a graph showing the results of analyzing the change in immunosuppressive activity according to the treatment order when hUCB-MSCs and pimecrolimus were treated in combination.

As shown in FIG. 5d, when hUCB-MSCs and pimecrolimus were treated simultaneously, no immunosuppressive activity was observed (experimental group 1), but when hUCB-MSCs and pimecrolimus were treated sequentially according to various combinations of treatment sequence orders, excellent immunosuppressive activity was observed (Experimental Groups 4 and 7).

In particular, when pimecrolimus was treated first, followed by hUCB-MSCs (Experimental Group 4), it showed relatively superior immunosuppressive activity compared to when hUCB-MSCs were treated first, followed by pimecrolimus (Experimental Group 7).

In summary of the results of the above analysis, it was confirmed that pimecrolimus and hUCB-MSCs, each of which exhibits immunosuppressive activity, may result in large differences in immunosuppressive activity depending on the combination treatment method. That is, according to the results of Example 3, it seemed that pimecrolimus unilaterally interfered with the immunosuppressive activity of hUCB-MSCs, but in reality, it was confirmed that hUCB-MSCs may also affect the immunosuppressive activity of pimecrolimus.

As a result, it was found that hUCB-MSCs and pimecrolimus can have a synergistic effect when treated in combination, but for this to happen, they must be treated sequentially rather than simultaneously. In particular, it was further confirmed that in the case of the sequential treatment above, the most superior immunosuppressive activity can be obtained when pimecrolimus was treated first, followed by hUCB-MSCs

## Claims

1. A pharmaceutical combination for use in preventing or treating an immune disease caused by excessive immunity, comprising separately:
(a) a first composition containing pimecrolimus or a pharmaceutically acceptable salt thereof; and
(b) a second composition containing mesenchymal stem cells,
wherein the first composition and the second composition are administered sequentially.

2. The pharmaceutical combination for use according to claim 1, wherein the mesenchymal stem cells are selected from the group consisting of adipose-derived mesenchymal stem cells, umbilical cord blood-derived mesenchymal stem cells, umbilical cord-derived mesenchymal stem cells, cardiac muscle-derived mesenchymal stem cells, placenta-derived mesenchymal stem cells, cartilage-derived mesenchymal stem cells, bone marrow-derived mesenchymal stem cells, and a combination thereof.

3. The pharmaceutical combination for use according to claim 1, wherein the immune disease caused by excessive immunity is an allergic immune disease or an autoimmune disease.

4. The pharmaceutical combination for use according to claim 3, wherein the allergic immune disease is selected from the group consisting of hay fever, allergic rhinitis, allergic conjunctivitis, atopic dermatitis, food allergy, mastocytosis, and a combination thereof.

5. The pharmaceutical combination for use according to claim 3, wherein the autoimmune disease is selected from the group consisting of Graves' disease, rheumatoid arthritis, Hashimoto's thyroiditis, systemic lupus erythematosus (lupus), vasculitis, Addison's disease, polymyositis, Sjögren's syndrome, progressive systemic sclerosis, and a combination thereof.

6. The pharmaceutical combination for use according to claim 1, wherein the first composition is first administered once or multiple times, and subsequently, the second composition is administered once or multiple times.

7. The pharmaceutical combination for use according to claim 6, wherein following the sequential administration of first composition and the second composition, the treatment further comprises additional administration of the first composition or the second composition individually once or multiple times.

8. The pharmaceutical combination for use according to claim 1, wherein the second composition is first administered once or multiple times, and subsequently, the first composition is administered once or multiple times.

9. The pharmaceutical combination for use according to claim 8, wherein following the sequential administration of second composition and the first composition, the treatment further comprises additional administration of the first composition or the second composition individually once or multiple times.

10. The pharmaceutical combination for use according to claim 1, further comprising a pharmaceutically acceptable carrier, excipient, or diluent.

## Patentansprüche

1. Pharmazeutische Kombination zur Verwendung beim Vorbeugen oder Behandeln einer Immunerkrankung, die durch übermäßige Immunität verursacht wird, getrennt umfassend:
(a) eine erste Zusammensetzung, die Pimecrolimus oder ein pharmazeutisch verträgliches Salz davon enthält; und
(b) eine zweite Zusammensetzung, die mesenchymale Stammzellen enthält,
wobei die erste Zusammensetzung und die zweite Zusammensetzung sequentiell verabreicht werden.

2. Pharmazeutische Kombination zur Verwendung nach Anspruch 1, wobei die mesenchymalen Stammzellen ausgewählt sind aus der Gruppe bestehend aus von Körperfett abgeleiteten mesenchymalen Stammzellen, von Nabelschnurblut abgeleiteten mesenchymalen Stammzellen, von Nabelschnur abgeleiteten mesenchymalen Stammzellen, von Herzmuskel abgeleiteten mesenchymalen Stammzellen, von Plazenta abgeleiteten mesenchymalen Stammzellen, von Knorpel abgeleiteten mesenchymalen Stammzellen, von Knochenmark abgeleiteten mesenchymalen Stammzellen und einer Kombination davon.

3. Pharmazeutische Kombination zur Verwendung nach Anspruch 1, wobei die Immunerkrankung, die durch übermäßige Immunität verursacht wird, eine allergische Immunerkrankung oder eine Autoimmunerkrankung ist.

4. Pharmazeutische Kombination zur Verwendung nach Anspruch 3, wobei die allergische Immunerkrankung ausgewählt ist aus der Gruppe bestehend aus Heuschnupfen, allergischer Rhinitis, allergischer Konjunktivitis, atopischer Dermatitis, Nahrungsmittelallergie, Mastozytose und einer Kombination davon.

5. Pharmazeutische Kombination zur Verwendung nach Anspruch 3, wobei die Autoimmunerkrankung ausgewählt ist aus der Gruppe bestehend aus Morbus Basedow, rheumatoider Arthritis, Hashimoto-Thyreoiditis, systemischem Lupus erythematodes (Lupus), Vaskulitis, Morbus Addison, Polymyositis, Sjögren-Syndrom, progressiver systemischer Sklerose und einer Kombination davon.

6. Pharmazeutische Kombination zur Verwendung nach Anspruch 1, wobei die erste Zusammensetzung zuerst einmal oder mehrmals verabreicht wird und anschließend die zweite Zusammensetzung einmal oder mehrmals verabreicht wird.

7. Pharmazeutische Kombination zur Verwendung nach Anspruch 6, wobei die Behandlung nach der sequentiellen Verabreichung der ersten Zusammensetzung und der zweiten Zusammensetzung ferner eine zusätzliche Verabreichung der ersten Zusammensetzung oder der zweiten Zusammensetzung einzeln einmal oder mehrmals umfasst.

8. Pharmazeutische Kombination zur Verwendung nach Anspruch 1, wobei die zweite Zusammensetzung zuerst einmal oder mehrmals verabreicht wird und anschließend die erste Zusammensetzung einmal oder mehrmals verabreicht wird.

9. Pharmazeutische Kombination zur Verwendung nach Anspruch 8, wobei die Behandlung nach der sequentiellen Verabreichung der zweiten Zusammensetzung und der ersten Zusammensetzung ferner eine zusätzliche Verabreichung der ersten Zusammensetzung oder der zweiten Zusammensetzung einzeln einmal oder mehrmals umfasst.

10. Pharmazeutische Kombination zur Verwendung nach Anspruch 1, ferner umfassend einen pharmazeutisch verträglichen Träger, Hilfsstoff oder Verdünner.

## Revendications

1. Combinaison pharmaceutique destinée à être utilisée dans la prévention ou le traitement d'une maladie immunitaire causée par une immunité excessive, comprenant séparément :
(a) une première composition contenant du pimécrolimus ou un sel pharmaceutiquement acceptable de celui-ci ; et
(b) une seconde composition contenant des cellules souches mésenchymateuses,
ladite première composition et ladite seconde composition étant administrées de manière séquentielle.

2. Combinaison pharmaceutique destinée à être utilisée selon la revendication 1, lesdites cellules souches mésenchymateuses étant choisies dans le groupe constitué par les cellules souches mésenchymateuses dérivées de tissus adipeux, les cellules souches mésenchymateuses dérivées de sang de cordon ombilical, les cellules souches mésenchymateuses dérivées de cordon ombilical, les cellules souches mésenchymateuses dérivées de muscle cardiaque, les cellules souches mésenchymateuses dérivées de placenta, les cellules souches mésenchymateuses dérivées de cartilage, les cellules souches mésenchymateuses dérivées de moelle osseuse et une combinaison de celles-ci.

3. Combinaison pharmaceutique destinée à être utilisée selon la revendication 1, ladite maladie immunitaire causée par une immunité excessive étant une maladie immunitaire allergique ou une maladie auto-immune.

4. Combinaison pharmaceutique destinée à être utilisée selon la revendication 3, ladite maladie immunitaire allergique étant choisie dans le groupe constitué par le rhume des foins, la rhinite allergique, la conjonctivite allergique, la dermatite atopique, une allergie alimentaire, la mastocytose, et une combinaison de ceux-ci.

5. Combinaison pharmaceutique destinée à être utilisée selon la revendication 3, ladite maladie auto-immune étant choisie dans le groupe constitué par la maladie de Basedow, la polyarthrite rhumatoïde, la thyroïdite de Hashimoto, le lupus érythémateux systémique (lupus), la vascularite, la maladie d'Addison, la polymyosite, le syndrome de Sjögren, la sclérose systémique progressive et une combinaison de ceux-ci.

6. Combinaison pharmaceutique destinée à être utilisée selon la revendication 1, ladite première composition étant d'abord administrée une ou plusieurs fois, et par la suite, ladite seconde composition étant administrée une ou plusieurs fois.

7. Combinaison pharmaceutique destinée à être utilisée selon la revendication 6, après ladite administration séquentielle de la première composition et de la seconde composition, ledit traitement comprenant en outre l'administration supplémentaire de la première composition ou de la seconde composition individuellement une ou plusieurs fois.

8. Combinaison pharmaceutique destinée à être utilisée selon la revendication 1, ladite seconde composition étant d'abord administrée une ou plusieurs fois, et par la suite, ladite première composition étant administrée une ou plusieurs fois.

9. Combinaison pharmaceutique destinée à être utilisée selon la revendication 8, après ladite administration séquentielle de la seconde composition et de la première composition, ledit traitement comprenant en outre l'administration supplémentaire de la première composition ou de la seconde composition individuellement une ou plusieurs fois.

10. Combinaison pharmaceutique destinée à être utilisée selon la revendication 1, comprenant en outre un vecteur, un excipient ou un diluant pharmaceutiquement acceptable.
